Europäisches Patentamt

European Patent Office

Office européen des brevets

(19)

(11) Publication number: **0 320 571**
**A2**

## (12) EUROPEAN PATENT APPLICATION

(21) Application number: 88113312.8

(51) Int. Cl.4: **C07C 2/30**

(22) Date of filing: 17.08.88

(30) Priority: 18.12.87 JP 318574/87

(43) Date of publication of application:
21.06.89 Bulletin 89/25

(84) Designated Contracting States:
BE CH DE FR GB IT LI NL SE

(71) Applicant: IDEMITSU PETROCHEMICAL CO.
LTD.
1-1, Marunouchi 3-chome Chiyoda-ku
Tokyo(JP)

(72) Inventor: Nakashima, Yoshitomi
IDEMITSU PETROCHEM.CO.,LTD. No. 1-1,
Miyamae-cho
Tokuyama-shi Yamaguchi-ken(JP)
Inventor: Takeuci, Kunio
IDEMITSU PETROCHEM.CO.,LTD. No. 1-1,
Miyamae-cho
Tokuyama-shi Yamaguchi-ken(JP)
Inventor: Fujita, Norio
IDEMITSU PETROCHEM.CO.,LTD. No. 1-1,
Miyamae-cho
Tokuyama-shi Yamaguchi-ken(JP)

(74) Representative: Türk, Dietmar, Dr. rer. nat. et
al
Türk, Gille + Hrabal Patentanwälte Bruckner
Strasse 20
D-4000 Düsseldorf 13(DE)

(54) Production of alpha-olefins.

(57) The invention provides a solution for the prob-
lem due to the by-product polymer in the process
for the production of α-olefin compounds by the
oligomerization reaction of ethylene in the presence
of a Ziegler catalyst. In the improved method ac-
cording to the invention, the reaction mixture after
completion of the reaction is subjected to an ad-
iabatic flashing treatment to recover the unreacted
ethylene and to precipitate the by-product polymer
which is crushed to have a specified particle diam-
eter and the reaction mixture containing the polymer
particles is heated after deactivation of the catalyst
and deashing up to a temperature sufficient to dis-
solve the polymer particles prior to isolation of the α-
olefin products by distillation.

# PRODUCTION OF α-OLEFINS

## BACKGROUND OF THE INVENTION

The present invention relates to an improvement in the production of α-olefins having 4 to 18 carbon atoms in a molecule by the oligomerization of ethylene. More particularly, the invention relates to an improvement in the production of α-olefins having usefulness as a starting material of various kinds of polymers, plasticizers, surface active agents and the like by the oligomerization of ethylene, according to which the process can be run with stability as being freed from the troubles such as clogging of the apparatus even without discharging the by-product polymer out of the reaction system.

As is well known, α-olefins are widely used in large quantities as a monomer or comonomer of polyolefins and various kinds of other copolymeric products and as a starting material of plasticizers, surface active agents and the like.

The α-olefin here implied is an ethylenically unsaturated compound having 4 to 18 carbon atoms in a molecule, which is an oligomer of ethylene and usually produced by the oligomerization of ethylene in a single-step process using a Zieglertype catalyst. Generally, the process comprises several steps including a step of the oligomerization reaction of ethylene to form α-olefins, a step for the recovery of unreacted ethylene, a step for the deactivation of the catalyst and deashing of the product and a step for the fractional distillation of the solvent and the respective compounds of the α-olefin product. The largest problem to enhance the productivity of the process is how to dispose the polymeric by-product having a viscosity-average molecular weight of as high as about one million.

Namely, the by-product polymer causes very serious troubles of clogging in the pipings, valves, orifices, heat exchangers, pumps, mixers and the like in the recovery line of the unreacted ethylene and the post-treatment line for the deactivation of the catalyst and deashing of the product resulting in an undesirable situation of the production system as a whole which can no longer be operated with stability. It is a conventional measure in the prior art that the by-product polymer is discharged out of the production line by using a filter and the like. Such a prior art method is, however, defective in respect of the unavoidably high investment for the facilities and the high cost of utilities required for the removal of the by-product polymer as well as in respect of the labor cost for discarding the removed by-product polymer.

## SUMMARY OF THE INVENTION

An object of the present invention is therefore to provide an improved method for the production of α-olefins which is freed from the above described problems and disadvantages in the prior art method for the production of α-olefins in a single-step process by the oligomerization of ethylene by using a Ziegler-type catalyst and capable of being operated with stability not to cause troubles such as clogging of the apparatuses even when the by-product polymer is not discharged out of the production line.

Thus, the improvement provided by the present invention comprises, in a method for the production of α-olefin compounds comprising the steps of oligomerizing ethylene in the presence of a Ziegler-type catalyst, recovering unreacted ethylene from the reaction mixture, subjecting the reaction mixture to a deactivation treatment of the catalyst and deashing treatment of the mixture and isolating the α-olefin compounds from the reaction mixture, crushing the polymer precipitated in the reaction mixture in the course of the recovery of the unreacted ethylene from the reaction mixture and heating the fraction containing the α-compounds and the crushed polymer particles to dissolve the polymer therein prior to the isolation of the compounds from the fraction.

## BRIEF DESCRIPTION OF THE DRAWING

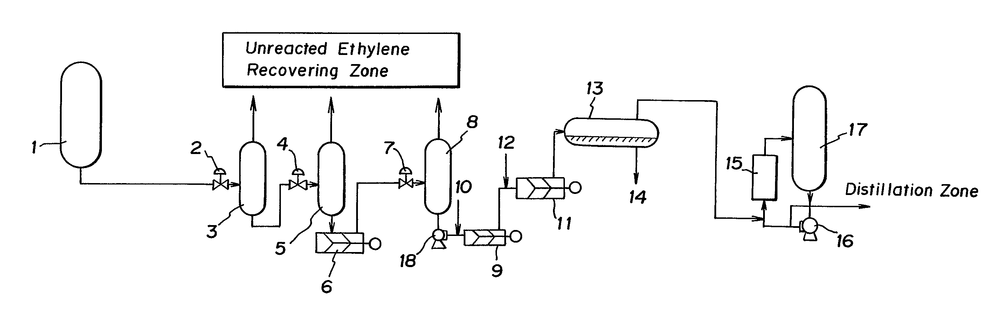

Figure 1 is a flowchart illustrating the improved process for the production of α-olefin compounds by the oligomerization of ethylene according to the present invention.

## DETAILED DESCRIPTION OF THE PREFERRED EMBODIMENTS

As is described above, the present invention has been completed on the base of the discovery unexpectedly obtained as a result of the extensive investigations undertaken by the inventors that the problems in the prior art methods for the production of α-olefin compounds by the oligomerization of ethylene can be solved by undertaking successive steps by subjecting the reaction mixture after the oligomerization reaction of ethylene to form α-olefin compounds to a treatment of adiabatic flashing to vaporize the unreacted ethylene dissolved therein with simultaneous precipitation of the polymer contained in the reaction mixture, crushing the precipitated polymer, subjecting the reaction mixture to a deactivation treatment of the catalyst and deashing treatment of the mixture, heating the reaction mixture to dissolve the crushed polymer and isolating and recovering the $\alpha$-olefin compounds from the reaction mixture.

In the method according to the improvement of the present invention, the $\alpha$-olefin compounds can be obtained by the oligomerization reaction of ethylene in the presence of a Ziegler-type catalyst. The Ziegler-type catalyst used in the oligomerization reaction of ethylene is composed of (A) a transition metal ingredient, (B) an organometallic ingredient and, optionally, a complex ligand in combination. The transition metal ingredient (A) is a compound represented by the general formula

$$ZX_aA_{4-a} \quad \ldots \text{(I)}$$

in which Z is an atom of zirconium or titanium, X and A, which can be the same ones or different ones from each other, are each an atom of halogen selected from the group consisting of chlorine, bromine and iodine, and the subscript a is zero or a positive integer not exceeding 4. Examples of the compounds suitable as the transition metal ingredient include $ZrCl_4$, $ZrBr_4$, $ZrI_4$, $ZrBrCl_3$, $ZrBr_2Cl_2$ $TiCl_4$, $TiBr_4$, $TiI_4$, $TiBrCl_3$, $TiBr_2Cl_2$ and the like.

The organometallic ingredient (B) in the Ziegler-type catalyst is a compound represented by the general formula

$$Al_2R_3Q_3 \quad \ldots \text{(II)}$$

in which R is an alkyl group having 1 to 20 carbon atoms and Q is an atom of halogen selected form the group consisting of chlorine, bromine and iodine. Alternatively, the organometallic ingredient (B) in the Ziegler-type catalyst may be a compound represented by the general formula

$$AlR^1_bQ^1_{3-b} \quad \ldots \text{III}$$

in which $R^1$ is an alkyl group having 1 to 20 carbon atoms, $Q^1$ is an atom of halogen selected form the group consisting of chlorine, bromine nad iodine and the subscript b is zero or a positive integer not exceeding 3.

Examples of the compound represented by the above given general formula (II) include $Al_2(CH_3)_3Cl_3$, $Al_2(CH_3)_3Br_3$, $Al_2(C_2H_5)_3Cl_3$, $Al_2(C_2H_5)_3Br_3$, $Al_2(C_2H_5)_3I_3$, $Al_2(C_2H_5)_3BrCl_2$, $Al_2(C_3H_7)_3Cl_3$, $Al_2(iso\text{-}C_3H_7)_3Cl_3$, $Al_2(C_4H_9)_3Cl_3$, $Al_2(iso\text{-}C_4H_9)_3Cl_3$, $Al_2(C_5H_{11})_3Cl_3$, $Al_2(C_8H_{17})_3Cl_3$, $Al_2(C_2H_5)_2(CH_3)Cl_3$ and the like.

Examples of the compound represented by the above given general formula (III) include $Al(CH_3)_3$, $Al(C_2H_5)_3$ $Al(C_3H_7)_3$, $Al(iso\text{-}C_3H_7)_3$, $Al(C_4H_9)_3$, $Al(iso\text{-}C_4H_9)_3$, $Al(C_5H_{11})_3$, $Al(C_6H_{13})_3$, $Al(C_8H_{17})_3$, $Al(C_2H_5)_2Cl$, $Al(C_2H_5)_2Br$, $Al(C_2H_5)_2I$, $Al(C_2H_5)Cl_2$, $Al(C_2H_5)Br_2$, $Al(C_2H_5)I_2$ and the like.

Further, the complex ligand used according to need as an ingredient of the catalyst system is a compound selected from the group consisting of sulfur compounds, phosphorus compounds and nitrogen compounds.

Although a variety of sulfur compounds can be used as the complex ligand above mentioned, examples of preferable sulfur compounds include thioether compounds such as dimethyl sulfide, diethyl sulfide, dipropyl sulfide, dihexyl sulfide, dicyclohexyl sulfide, diphenyl thioether and the like, dialkyl disulfide compounds such as dimethyl disulfide, diethyl disulfide, dipropyl disulfide, dibutyl disulfide, dihexyl disulfide, dicyclohexyl disulfide, ehtyl methyl disulfide and the like, thiophene compounds such as thiophene, 2-methyl thiophene, 3-methyl thiophenen, 2,3-dimethyl thiopnene, 2-ethyl thiophene, benzothiophene and the like, hetero cyclic sulfur compounds such as tetrahydro thiophene, thiopyrane and the like, aromatic sulfide compounds such as diphenyl sulfide, diphenyl disulfide, methyl phenyl disulfide, methyl phenyl sulfide and the like, thiourea, sulfide compounds such as methyl sulfide, ethyl sulfide, butyl sulfide and the like, and so on.

Although a variety of phosphorus compounds can be used as the complex ligand above mentioned, examples of preferable phosphorus compounds include phosphine compounds such as triphenyl phosphine, triethyl phosphine, tributyl phosphine, tripropyl phosphine, trioctyl phosphine, tricyclohexyl phosphine and the like.

Although a variety of nitrogen compounds can be used as the complex ligand above mentioned, examples of preferable nitrogen compounds include organic amine compounds such as methyl amine, ethyl amine, propyl amine, butyl amine, pentyl amine, hexyl amine, cyclohexyl amine, octyl amine, decyl amine, aniline, benzyl amine, naphthyl amine, dimethyl amine, diethyl amine, dibutyl amine, diphenyl amine, methyl phenyl amine, trimethyl amine, triethyl amine, tributyl amine, triphenyl amine, pyridine, picoline and the like.

The catalyst composed of (A) the transition metal ingredient and (B) the organometallic ingredient can be prepared in various methods without particular limitation. It is, however, preferable in order to prepare a catalyst system having high catalytic activity and capable of giving an $\alpha$-olefin product having an advantageous molecular weight distribution that the organometallic compound represented by the general formula (III) such as $Al(C_2H_5)_3$ is first introduced and then the transition metal compound such as $ZrCl_4$ is introduced followed by the introduction of the organometallic compound represented by the general formula (II) such as $Al_2(C_2H_5)_3Cl_3$ or, though less preferable,

that the introduction of the transition metal compound follows the introduction of the organometallic compounds represented by the general formulae (II) and (III). In this case, the order of the introduction of the organometallic compounds represented by the general formulae (II) and (III) is not limitative.

The oligomerization reaction of ethylene by using the catalyst prepared in the above described manner is performed usually in an organic solvent. Examples of organic solvents suitable for this purpose include naphthenic paraffin hydrocarbons such as cyclohexane, decahydronaphthalene and the like, aromatic hydrocarbons and halogenated derivatives thereof such as benzene, toluene, xylene, chlorobenzene, ethyl benzene, dichlorobenzene, chlorotoluene and the like, aliphatic hydrocarbons such as pentane, hexane, heptane, octane, nonane, decane and the like and halogenated alkanes such as dichloroethane, dichlorobutane and the like.

The oligomerizaiton reaction of ethylene according to the improvement of the present invention is carried out usually at a temperature in the range from 100 to 130°C under a pressure in the range from 30 to 70 kg/cm²G. The reaction is complete usually within a reaction time of 10 to 60 minutes although the exact length of the reaction time should be dependent on the reaction parameters such as the temperature and pressure.

In the production of α-olefin compounds by the oligomerization of ethylene in the method improved according to the invention, the reaction mixture after completion of the oligomerization reaction is first subjected to a treatment of adiabatic flashing so as to vaporize the unreacted ethylene dissolved therein and to precipitate the polymer produced as a by-product. The by-product polymer is crystalline and the viscosity-average molecular weight thereof is usually about one million. The adiabatic flashing of the reaction mixture is usually repeated several times. The flowchart of the accompanying drawing illustrates a process in which the adiabatic flashing treatment is repeated three times.

In the next place, the by-product polymer precipitated in the reaction mixture is crushed or broken by using a suitable machine such as a shearing cutter and then the reaction mixture is transferred to the process for the deactivation of the catalyst and deashing of the reaction mixture. Although the crushing treatment of the precipitated by-product polymer is undertaken after the flashing treatment of the mixture, it is desirable that, when the flashing treatment is repeated three times, crushing of the precipitated polymer is undertaken after each of the second and the third flashing treatments. By this crushing treatment of the precipitated polymer, the particle size of the polymer is decreased, preferably, to 1000 μm or smaller.

The method for the deactivation of the catalyst and deashing is not particularly limitative and any known method conventionally used in the production of α-olefin compounds from ethylene can be applied. For example, the reaction mixture is admixed with an amine compound as a deactivating agent and then washed with water to effect the deactivation treatment of the catalyst and the deashing treatment. The deactivation treatment of the catalyst can be undertaken concurrently with the crushing treatment of the precipitated polymer after the third stage of the adiabatic flashing.

In the next place, the reaction mixture after the treatment in the above described manner is transferred to the process for the isolation of the α-olefin compounds. In this process, the reaction mixture after washing with water was subjected to phase separation into the aqueous phase and the organic phase containing the α-olefin compounds and the crushed polymer and the thus separated organic fraction is heated up to a temperature of, usually, 90 to 130°C so as to rapidly dissolve the polymer in the mixture. In particular, the organic fraction at a temperature of 40 to 50°C is mixed with the reaction mixture at a temperature of 120 to 130°C and the mixture is introduced into a heat exchanger where it is heated up to a temperature of about 120 to 130°C. The reaction mixture, in which the polymer has been dissolved, is then subjected to distillation to isolate the α-olefin compounds along with recovery of the solvent. The unreacted ethylene and the solvent recovered in this manner can be recycled to the subsequent runs of the reaction. The product obtained by distillation is a mixture of various kinds of α-olefin compounds having 4 to 18 carbon atoms in a molecule formed by the oligomerization reaction of ethylene and can be separated into the respective α-olefin compounds of high purity by subjecting the mixture to multistage rectification distillation. It is of course possible to obtain a particular species of the α-olefin compounds in an increased yield at the sacrifice of the other species of the α-olefin compounds according to desire by appropriately selecting the conditions of the oligomerization reaction.

In the following, the method according to the improvement of the present invention is described in more detail with reference to the accompanying drawing, of which Figure 1 illustrates a flowchart of a preferred process for the production of α-olefin compounds by the oligomerization reaction of ethylene. The reaction mixture prepared in the reaction vessel 1 and containing a Ziegler-type catalyst, solvent, unreacted ethylene and the α-olefin compounds as the product is transferred through the control valve 2 into the first-stage flashing tank 3 and then through the control valve 4 into the second-stage flashing tank 5. Most of the unreacted ethylene dissolved in the reaction mixture is vaporized in these flashing tanks to be recovered along with precipitation of the by-product polymer in the reaction mixture staying in the second-stage flashing tank 5. After a very short staying time in the second-stage flashing tank 5, the reaction mixture containing the precipitated polymer is transferred to the shearing cutter 6 installed below the bottom of the second-stage flashing tank 5, where the precipitated polymer is subjected to the crushing treatment so as to have a particle size of $1000\mu m$ or smaller. The reaction mixture containing the polymer particles is then transferred through the control valve 7 into the third-stage flashing tank 8 where it is almost completely freed from the unreacted ethylene.

In the next place, the reaction mixture containing the thus crushed polymer is transferred into the shearing cutter 9 where the polymer is again crushed to have a particle size of 1000 $\mu$m or smaller. Deactivation treatment of the catalyst is undertaken concurrently in the shearing cutter 9 by introducing a deactivating agent through the inlet port 10. Thereafter, the reaction mixture is admixed with water for washing and transferred into the deashing tank 11 to effect deashing. The reaction mixture after the deashing treatment is transferred to the phase separator 13 where it is separated into the organic phase and the aqueous phase. The aqueous solution obtained by the phase separation is discarded as a waste. The organic solution containing the $\alpha$-olefin compounds and the polymer particles, which is usually at a temperature of 40 to $50\degree$C, is mixed with the reaction mixture supplied by means of the pump 16, which is at a high temperature of 120 to $130\degree$C, to be heated at about $100\degree$C so as to rapidly dissolve the polymer particles contained therein and introduced into the heat exchanger 15 where it is further heated up to a temperature of 120 to $130\degree$C before introduction into the reservoir tank 17. The reaction mixtrue at a high temperature obtained in this manner is then transferred to the process of distillation and separated into the solvent and the $\alpha$-olefin compounds, and furhter the by-product polymer is removed.

According to the improved method of the present invention, $\alpha$-olefin compounds can be produced by the oligomerization reaction of ethylene by utilizing a Ziegler-type catalyst without troubles to disturb stable running of the production facilities such as clogging of the apparatus even without removing the by-product polymer out of the production line in the course of the reaction.

The $\alpha$-olefin compounds produced by the method according to the improvement of the present invention are useful as a monomer of polyolefins or a comonomer of various kinds of copolymeric resins or as a starting material of various kinds of plasticizers, surface active agents and the like.

In the following, the improvement according to the present invention is described in more detail by way of examples, which should not be construed to limit the scope of the invention in any way.

Example 1.

The Ziegler-type catalyst used in this example was composed of $ZrCl_4$, $Al_2(C_2H_5)_3Cl_3$ and $Al(C_2H_5)_3$ in such a molar ratio that the ratio of $[ZrCl_4]:[Al_2(C_2H_5)_3Cl_3 + Al(C_2H_5)_3]$ was 1:7 and the ratio of $[Al_2(C_2H_5)_3Cl_3]:[Al(C_2H_5)_3]$ was 3.5:1.

The above described zirconium tetrachloride/alkyl aluminumbased catalyst was added to cyclohexane in an amount of 0.15 m mole per liter of the solvent and the cyclohexane solution was used as the reaction medium for the oligomerization reaction of ethylene which was carried out for 40 minutes at a temperature of $120\degree$C under a pressure of 60 $kg/cm^2G$ to produce $\alpha$-olefin compounds. The reaction mixture after completion of the reaction contained about 0.05% by weight of a polymer as a by-product dissolved therein which had a viscosity-average molecular weight of about one million.

The reaction mixture was subjected to a post-treatment according to the flowchart illustrated in Figure 1. Thus, the reaction mixture was first subjected to an adiabatic flashing treatment to evaporate the unreacted ethylene dissolved therein so that precipitation of the by-product polymer was started in the second-stage flashing tank 5 at a moment when the temperature of the reaction mixture had decreased to 80 to $90\degree$C. The precipitated polymer particles had a particle diameter distribution in the range from the sub-micron order to several tens of mm. The reaction mixture was then transferred into the shearing cutter 6 in which the polymer particles were crushed to have a particle diameter not exceeding 1000 $\mu$m. The reaction mixture was transferred into the third-stage flashing tank 8 in which it was freed completely from the unreacted ethylene dissolved therein. The reaction mixture was admixed in the third-stage flashing tank 8 with ammonia water of 20 to 50% by weight concentration as a deactivating agent in a volume of 20 to 100 ml per liter of the reaction mixture and transferred into the second shearing cutter 9 where it was agitated to deactivate the catalyst and the polymer particles were again crushed to have a particle diameter not exceeding 1000 um. Therefore, the reaction mixture was subjected to a deashing treatment by washing with water. The reaction mixture freed from the aqueous phase in the phase separator 13 was at a temperature of 40 to 50°C and the by-product polymer contained therein was solid. The reaction mixture was then admixed with a fresh portion of the reaction mixture at 120 to 130°C coming from the reservoir tank 17 so that the temperature of the reaction mixture was increased to 90 to 100°C and the polymer was rapidly dissolved therein. The reaction mixture containing the thus dissolved polymer was introduced into the heat exchanger 15 in which it was reheated up to 120 to 130°C and then transferred to the reservoir tank 17. The reaction mixture in the reservoir tank 17 was transferred to the process of distillation to isolate the α-olefin compounds and the solvent and to recover the by-product polymer as a residue. The thus obtained product was composed mainly of α-olefin compounds having 4 to 18 carbon atoms in a molecule.

In the above described process for the production of α-olefin compounds from ethylene, all of the procedures could be conducted very smoothly without troubles due to clogging of the apparatuses and pipings with the polymer. The control valves 2, 4 and 7 were each of the type of an angle valve which could effectively prevent deposition of the polymer and facilitate falling of the deposited polymer.

Comparative Example 1.

The conditions of the process were substantially the same as in the above described Example 1 except that no treatment was undertaken of the by-product polymer precipitated in the reaction mixture in the course of recovery of the unreacted ethylene by the adiabatic flashing of the reaction mixture after completion of the oligomerization reaction of ethylene.

The results were that blockage of the piping took place due to the growth of the precipitated polymer in a fibrous form on the control valves 4 and 7. In addition, seizure took place in the mechanical seals and rotatory parts of the pumps due to deposition of the polymer. Accordingly, the process could not be continued after about a week.

Comparative Example 2.

The conditions of the process were substantially the same as in Example 1 described above excepting omission of heating of the reaction mixture containing the polymer particles after the crushing treatment of the by-product polymer as precipitated in the shearing cutter.

The results were that no smooth running of the process could be conducted due to occasional blockage of the piping, especially, under fluctuation in the flow rate as a consequence of deposition of the polymer in the heat exchangers, stagnant-flow portions of the pipings, etc.

## Claims

1. A method for the production of α-olefin compounds by the oligomerization reaction of ethylene which comprises the steps of:

(a) oligomerizing ethylene in a reaction mixture containing a Ziegler-type catalyst to form α-olefin compounds and a polymer as a by-product;

(b) recovering unreacted ethylene from the reaction mixture to cause precipitation of the by-product polymer in the reaction mixture;

(c) subjecting the reaction mixture containing the precipitated polymer to a deactivation treatment of the catalyst and a deashing treatment by washing with water;

(d) crushing the precipitated by-product polymer into particles;

e) heating the reaction mixture containing the crushed polymer particles to such an extent that the polymer particles are dissolved in the reaction mixture; and

(f) isolating the α-olefin compounds from the reaction mixture containing the polymer dissolved therein.

2. The method as claimed in claim 1 wherein recovering of the unreacted ethylene in step (b) is carried out by subjecting the reaction mixture to an adiabatic flashing treatment.

3. The method as claimed in claim 1 wherein the crushed polymer particles in step (d) has a particle diameter not exceeding 1000 μm.

4. The method as claimed in claim 2 wherein the adiabatic flashing treatment is repeated at least three times.

5. The method as claimed in claim 3 wherein crushing of the precipitated polymer is undertaken twice after the second and the third adiabatic flashing treatments.

6. The method as claimed in claim 1 wherein the reaction mixture is heated in step (e) to a temperature in the range from 90°C to 130°C.

Unreacted Ethylene
Recovering Zone

Distillation Zone

EP 0 320 571 A2